Europäisches Patentamt

European Patent Office

Office européen des brevets

(19)

(11) Veröffentlichungsnummer: **0 402 735**

**A1**

# (12) EUROPÄISCHE PATENTANMELDUNG

(21) Anmeldenummer: **90110575.9**

(22) Anmeldetag: **05.06.90**

(51) Int. Cl.⁵: **B01F 15/00, B01F 13/06**

(30) Priorität: **15.06.89 DE 3919534**

(43) Veröffentlichungstag der Anmeldung:
**19.12.90 Patentblatt 90/51**

(84) Benannte Vertragsstaaten:
**AT CH DE ES FR GB IT LI**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Planck, Heinrich, Dr.**
**Weinbergstrasse 66**
**D-7440 Nürtingen 10(DE)**
Erfinder: **Elser, Christoph**
**Hindenburgerstrasse 82**
**D-7310 Plochingen(DE)**
Erfinder: **Grieben, Albrecht, Dr.**
**Frankensteiner Strasse 106**
**D-6100 Darmstadt(DE)**
Erfinder: **Ege, Werner, Dr.**
**Mailänder Strasse 18**
**D-6000 Frankfurt 70(DE)**

(54) **Verfahren und Einrichtung zum Vorbereiten von Knochenzement.**

(57) Verfahren zum Vorbereiten von Knochenzement für das nachfolgende Einbringen in die der Aufnahme einer Prothese dienende Höhlung eines Knochens. Die der Herstellung des Knochenzementes dienenden Komponenten werden in eine Kartusche eingebracht und während einer Mischphase miteinander vermischt und anschließend wird noch eine Ruhephase eingelegt. Die Mischphase und/oder die Ruhephase werden automatisch programmgesteuert und/oder prozeßgesteuert.

Fig.1

EP 0 402 735 A1

## Verfahren und Einrichtung zum Vorbereiten von Knochenzement

Die Erfindung betrifft ein Verfahren zum Vorbereiten von Knochenzement gemäß dem Oberbegriff des Anspruches 1 und eine Einrichtung zur Durchführung dieses Verfahrens.

Knochenzement dient dem Befestigen von Prothesen in Knochen menschlicher und tierischer Körper. Eine gute und dauerhafte Verbindung des Knochenzementes mit einer Prothese und dem Knochen setzt voraus, daß der Knochenzement vor dem Einbringen in die der Aufnahme der Prothese dienende Höhlung des betreffenden Knochens einwandfrei vorbereitet wurde. Dies bedeutet insbesondere, daß zu Beginn des Einbringens des Knochenzementes in die Knochenhöhlung der Polymerisationszustand des Knochenzementes so ist, daß die nunmehr erfolgende Verarbeitung des Knochenzementes zu möglichst guter, dauerhafter Verankerung der Prothese in der Knochenhöhlung führt, wobei auch die Verarbeitungszeit voll ausreichend sein soll, das heißt die Zeit, die dem Chirurgen zur Verfügung steht, um ab Beginn des Einbringens des Knochenzementes in die Knochenhöhlung alle für die Verankerung der Prothese in der richtigen Stellung in der Knochenhöhlung erforderlichen Arbeiten auszuführen, bevor die dann keine Veränderung der Stellung der Prothese mehr zulassende Aushärtungsphase des Knochenzementes beginnt.

Knochenzement wird im allgemeinen durch Mischen von flüssigem Methylmethacrylat-Monomer und pulverförmigem Polymethylmethacrylat gegebenfalls unter Zugabe von Härtern und Beschleunigern hergestellt. Jedoch kann der Knochenzement auch aus irgendwelchen anderen geeigneten Komponenten hergestellt werden.

Es ist eine Aufgabe der Erfindung, besonders gute Reproduzierbarkeit des Polymerisationszustandes des Knochenzementes am Ende der Ruhephase zu erzielen, wobei sich dieses Verfahren bei beliebigen, aus mindestens je einer pulverförmigen und flüssigen Komponente herstellbaren, polymerisierbaren Knochenzementen jeweils auf dem betreffendem Knochenzement abgestimmt durchführen lassen soll.

Diese Aufgabe wird erfindungsgemäß durch das in Anspruch 1 angegebene Verfahren gelöst. Eine erfindungsgemäße Einrichtung zur Durchführung dieses Verfahrens ist in Anspruch 16 angegeben.

Bei dem erfindungsgemäßen Verfahren wird der Ablauf der Mischphase und der nachfolgenden Ruhephase, während dem der Knochenzement aus seinen Komponenten hergestellt und für das nachfolgende Einbringen in eine Knochenhöhlung vorbereitet wird, selbsttätig programmgesteuert und/oder prozessgesteuert durchgeführt. Dies ermöglicht es, besonders gute Reproduzierbarkeit eines am Ende der Ruhephase für den betreffenden Knochenzement gewünschten Polymerisationszustandes unbeeinflußt von menschlichen Unzulänglichkeiten zu erreichen. Der Zustand des Knochenzements wird also durch das erfindungsgemäße Verfahren dem Operateur in reproduzierbarer Qualität zur Verfügung gestellt. Dies ermöglicht eine Optimierung der Zementfixierung der Prothese.

Unter programmgesteuert ist verstanden, daß ein auf den betreffenden Knochenzement abgestimmtes Programm vorgesehen ist, das auf einem Programmträger gespeichert sein kann und/oder ganz oder teilweise durch einen Rechner für den jeweiligen Knochenzement und seine Menge aus einzugebenden und/oder gespeicherten Daten und/oder Meßwerten und dergl. berechnet werden kann, das während der Mischphase und/oder der Ruhephase mindestens einen Programmschritt bei der Vorbereitumg des Knochenzementes steuert. Der einzelne Programmschritt kann ein starrer, nicht mehr beeinflußbarer Programmschritt oder ein von mindestens einem Parameter abhängiger, bspw. durch einen Rechner in Abhängigkeit des betreffenden mindestens einen Parameters berechenbarer Programmschritt sein.

Unter prozeßgesteuert ist verstanden, daß der während der Mischphase und/oder Ruhephase stattfindende Vorbereitungsprozess des Knochenzementes für dessen nachfolgende Verarbeitung mindestens einen mittels eines Fühlers oder Meßwertgebers erfassten Meßwert und/oder mindestens ein sonstiges Signal liefert, der bzw. das dazu benutzt wird, diesen Vorbereitungsprozeß, den man auch als Herstellungsprozeß bezeichnen kann, zu steuern oder, falls auch Programmsteuerung vorgesehen ist, noch zusätzlich zu der Programmsteuerung mit zu steuern.

Dies sei anhand von einigen Beispielen noch näher erläutert.

Wenn während der Herstellung des Knochenzementes die in dem betreffenden Operationssaal herrschende Raumtemperatur für die zeitliche Länge mindestens eines Programmschrittes, bspw. für die Zeitdauer des Verrührens der Knochenzementkomponenten miteinander und/oder für die Zeitlänge der Ruhephase von bekanntem Einfluß ist, dann kann man vorsehen, die Zeitlänge des betreffenden Programmschrittes in Abhängigkeit der einen Parameter bildenden Raumtemperatur zu berechnen und den berechneten Wert in die Programmsteuervorrichtung einzugeben.

Das Programm wird also dann in Abhängigkeit der Raumtemperatur variiert. Dies ist noch keine Prozeßsteuerung, sondern eine temperaturabhängige Programmsteuerung. Wenn also bspw. die Zeitdauer der Mischphase wie auch die Zeitdauer der Ruhephase in Abhängigkeit der Raumtemperatur für den betreffenden Knochenzement festgelegt ist, berechnet ein Rechner die Zeitlängen dieser Programmschritte oder es kann ein Datenfeld oder ein Kennlinienfeld, das diesen Parameter berücksichtigt, gespeichert sein und die Programmsteuervorrichtung sucht sich dann in Abhängigkeit der ihr gemeldeten Raumtemperatur das für diese Raumtemperatur geltende Programm aus dem Speicher aus bzw. berechnet es unter Verwendung der betreffenden, im Speicher gespeicherten Daten.

Ein Parameter kann auch die Menge des herzustellenden Knochenzementes sein, also seines Volumens oder seines Gewichtes und das Programm wird dann entsprechend der jeweiligen Menge, die von Hand oder mittels eines durch ein Lesegerät lesbaren Datenträgers in einen Rechner eingegeben werden kann, in Abhängigkeit dieses Parameters ebenfalls oder allein in Abhängigkeit dieses Parameters variiert werden.

Desgleichen kann vorgesehen sein, daß in den Rechner oder in einen ihm zugeordneten Speicher für die Prozeßsteuerung und/oder für die Programmsteuerung bzw. für die Aufstellung des jeweiligen Programms erforderliche Daten über eine Mehrzahl von unterschiedlichen Knochenzementen eingegeben sind, und es wird dann der jeweilige Knochenzementtyp in den Rechner eingegeben, der dann für diesen Knochenzement den oder die betreffenden Programmschritte und/oder Prozeßschritte unter Berücksichtigung des oder der vorliegenden Parameter und/oder Meßwerte berechnet.

Wenn eine Prozeßsteuerung vorgesehen ist, werden einem Rechner oder einer Signalverarbeitungsvorrichtung während der Vorbereitung des Knochenzementes Daten über diesen Vorbereitungsprozeß geliefert, beispielsweise die durch einen Temperaturfühler gefühlte Eigentemperatur der in der Kartusche enthaltenen Masse aus den Komponenten des Knochenzementes. Dann wird je nach dieser gefühlten Temperatur oder in Abhängigkeit dieser Temperatur beispielsweise die Zeitdauer der Mischphase und/oder die Zeitdauer der Ruhephase eingestellt bzw. verändert, beispielsweise um so kürzer vorgesehen, je höher diese Temperatur ist, was während des Vorbereitungprozesses stattfindet. Oder es wird der Polymerisationsgrad des Knochenzementes während der Ruhephase ermittelt und bei Erreichen eines vorbestimmten Polymerisationsgrades die Ruhephase beendet.

Durch die Erfindung gelingt es, beliebigen Knochenzement selbsttätig und reproduzierbar vorzubereiten, so daß er am Ende der Ruhephase reproduzierbar den vom Operateur gewünschten Polymerisationszustand erreicht hat, so daß der Operateur den Knochenzement in optimalem Zustand in die Knochenhöhlung einbringen und die Prothese in ihr befestigen kann.

Für eine Prozeßsteuerung kann noch vorgesehen sein, anstatt die Temperatur der in der Kartusche befindlichen Masse zu fühlen, die oberhalb der Masse in der Kartusche herrschende Temperatur oder eine Eigentemperatur der Kartusche zu fühlen.

Besonders zweckmäßig kann vorgesehen sein, daß der Innenraum der Kartusche zumindest während der Mischphase gegenüber der Umgebungsatmosphäre abgesperrt und dabei zumindest zeitweise, vorzugsweise ständig, an eine Unterdruckquelle angeschlossen wird. Dies dient dazu, daß der Knochenzement am Ende der Mischphase bzw. am Ende der Ruhephase möglichst keine Lufteinschlüsse enthält, was seine Festigkeit und Lebensdauer in der Knochenhöhlung verbessert. An sich ist es bereits bekannt, während der Mischphase in der Kartusche Unterdruck vorzusehen (EP-A2-0 178 658), doch ist dies ganz besonders vorteilhaft in Verbindung mit dem erfindungsgemäßen Verfahren, weil hierdurch auch die Reproduzierbarkeit des Zustandes des Knochenzementes am Ende der Ruhephase noch weiter verbessert wird. Dadurch wird eine Standardisierung des Knochenzementzustandes und damit auch des Zementiervorganges ermöglicht.

Ferner ist es für die Durchführung der Mischphase besonders zweckmäßig und desgleichen für die Reproduzierbarkeit der Knochenzementvorbereitung, wenn das rührwirksame Rührorgan für das Mischen während des Vermischens der Knochenzementkomponenten vollständig in die in der Kartusche befindliche Masse aus den Knochenzementkompenenten eingetaucht wird und/oder während des Mischens ständig oder zeitweise lageverstellt wird, vorzugsweise in der Masse auf und ab bewegt wird. Auch kann zu demselben Zweck besonders vorteilhaft vorgesehen sein, daß als rührwirksames Organ ein Flügelrad in Art eines Propellers verwendet wird.

Die Vorbereitung des Knochenzementes läßt sich weiterhin dadurch noch weiter verbessern, insbesondere die Reproduzierbarkeit des Polymerisationszustandes des Knochenzementes am Ende der Ruhephase, indem die Knochenzementkomponenten zu Beginn der Mischphase auf vorbestimmte niedrige Temperaturen abgekühlt sind. Eine Kühlung auf Temperaturen von 0 bis 10° C, vorzugsweise 4 bis 8° C, empfiehlt sich besonders für die sogenannten "Standard Viscosity" Knochenzemente, die bei Raumtemperatur nur sehr schwer im Vakuum angerührt werden können.

Infolge dieser niedrigen Temperaturen der Knochenzementkomponenten am Beginn der Mischphase erfolgt das Polymerisieren des Knochenzementes langsamer und man kann deshalb die Mischphase und auch die Ruhephase zeitlich verlängern und damit auch bessere Reproduzierbarkeit des Knochenzementes am Ende der Ruhephase erreichen.

Auch kann in vielen Fällen zweckmäßig vorgesehen sein, daß die Kartusche während der Mischphase und/oder während der Ruhephase zumindest zeitweise erwärmt und/oder gekühlt wird, derart, daß die Polymerisationsgeschwindigkeit des Knochenzementes hierdurch in vorbestimmter Weise beeinflusst, bspw. verlangsamt wird und/oder der Knochenzement am Ende der Ruhephase eine vorbestimmte Verarbeitungstemperatur hat, die vorzugsweise niedriger als die Raumtemperatur des betreffenden Operationssaales ist. Der Operateur weiß dann genau, welche Verarbeitungszeit ihm für das Einzementieren der Prothese in der betreffenden Knochenhöhlung zur Verfügung steht und es wird so die Möglichkeit von Zementierungsfehlern ausgeschlossen oder reduziert. Wenn, wie bevorzugt vorgesehen, die Temperatur des Knochenzementes am Ende der Ruhephase unter der Raumtemperatur des betreffenden Operationssaales liegt, vorzugsweise erheblich darunter liegt, beispielsweise 5 bis 20 Kelvin, dann werden Fehlerquellen verringert und genaueres Implantieren ermöglicht.

In der Zeichnung sind Ausführungsbeispiele der Erfindung dargestellt. Es zeigen:

Fig. 1 eine Vorderansicht einer Maschine zum Vorbereiten von Knochenzement, wobei eine Kartusche, ein sie tragender Schlitten und ein Rührorgan mit zugeordneter Welle, die alle vorzugsweise Einweg-Artikel sind, strichpunktiert dargestellt sind,

Fig. 2 eine Seitenansicht der Maschine nach Fig. 1,

Fig. 3 eine Draufsicht auf den Drehtisch der Maschine nach Fig. 1 und 2,

Fig. 4 in gebrochener Darstellung einen Längsschnitt durch eine am Drehtisch der Maschine nach Fig. 1 gehaltenen Kartusche, auf welche ein Deckel mittels eines plattenförmigen Druckteiles der Maschine nach den Fig. 1 und 2 angedrückt ist,

Fig. 5 eine Seitenansicht eines zweiten Ausführungsbeispieles einer Maschine zum Vorbereiten von Knochenzement,

Fig. 6 eine Abwandlung einer Einzelheit der Maschine nach Fig. 5 in teilweise geschnittener Darstellung,

Fig. 7 in geschnittener Darstellung zwei Ampullen, die die flüssige und die pulvrige Komponente eines herzustellenden Knochenzementes enthalten, wobei strichpunktiert dargestellt ist, wie diese ein Monomer bildende oder enthaltende Flüssigkeit und das Pulver einwandfrei septisch in die Kartusche, in welcher dieser Knochenzement herzustellen ist, bspw. auf der Maschine nach Fig. 5, eingefüllt werden können.

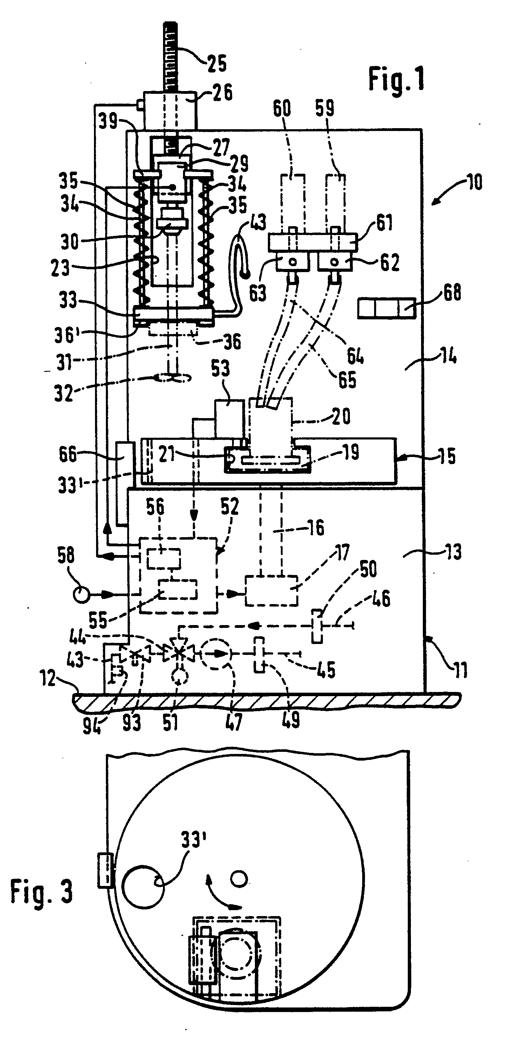

Die in den Fig. 1 bis 4 dargestellte Maschine 10, die man auch als Gerät oder Vorrichtung bezeichnen kann, dient dem Vorbereiten von Knochenzement für das nachfolgende Einbringen in eine der Aufnahme einer Prothese dienende Höhlung eines Knochens eines Menschen oder ggfl. auch eines Tieres. Sie weist ein Gestell 11 auf, an dem alle ihre Komponenten angeordnet sind. Im weiteren sei sie kurz als Maschine bezeichnet. Es ist selbstverständlich auch möglich, die eine oder andere ihrer Komponenten, bspw. ihre Programmsteuer- und/oder Prozeßsteuervorrichtung nicht an dem Gestell 11 anzuordnen, sondern bspw. an einem Bedienungspult, das von dem Gestell 11 getrennt angeordnet und mit diesem über elektrische und ggfs. sonstige flexible oder nicht flexible Leitungen verbunden ist.

Das Gestell 11 weist einen auf dem Boden 12 eines Operationssaales aufgestellten Kasten 13 auf, auf dessen Oberseite ein vertikaler Ständer 14 am rückwärtigen Ende des Kastens 13 fest angeordnet ist.

Auf der Oberseite des Kastens 13 ist ein um eine vertikale Drehachse beschränkt drehbarer Drehtisch 15 angeordnet, dessen Welle 16 in nicht näher dargestellter Weise im Kasten 13 drehbar gelagert und durch einen Antriebsmotor 17 zwischen zwei Positionen winkelverstellbar ist. Die eine Position, die in den Fig. 1 und 3 dargestellt ist, sei als Grundstellung bezeichnet und dient dem Einführen und wieder Entnehmen eines Schlittens 19 in eine in Bezug auf die Drehachse des Drehtisches radiale Geradführung 21, die in dem Drehtisch 15 angeordnet ist.

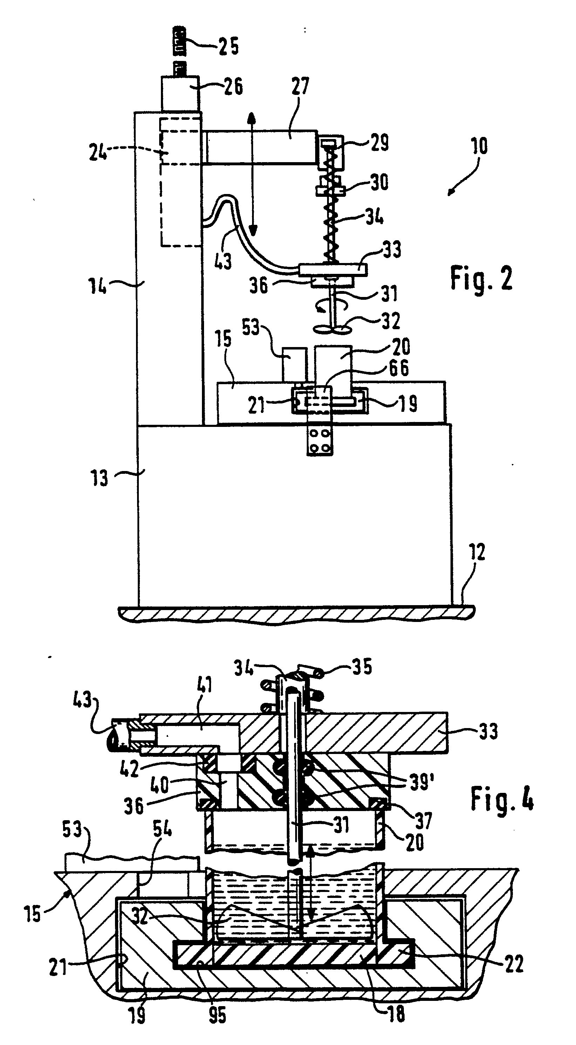

In diesen Schlitten 19 wird vor oder nach seinem unter Berücksichtigung der Sterilität erfolgendem Einsetzen in die Geradführung 21 eine Kartusche 20 eingesetzt und gehalten. Zu diesem Zweck weist die Kartusche 20, die bspw. aus opakem Kunststoff bestehen kann, untenseitig einen Ringflansch 22 (Fig. 4) auf, mit dem sie in eine eine Aufnahme für die Kartusche 20 bildende Geradführung 95 im Schlitten 19, wie besonders deutlich aus Fig. 4 zu ersehen ist, eingeschoben ist, und zwar bis in eine durch einen Anschlag begrenzte Endstellung. Die Kartusche 20, die einen für das spätere Herauspressen des vorbereiteten Knochenzementes dienenden, als Kolben wirkenden, axial beweglichen Boden 18 aufweist, ragt mit ihrer vorzugsweise kreiszylindrischen Umfangswandung durch eine Ausnehmung des Schlittens 19 und einen Schlitz des Drehtisches 15 nach oben über den Drehtisch 15 hinaus.

Am Ständer 14 ist eine vertikale Geradführung 23 angeordnet, in welcher ein Schlitten 24 bspw. mittels einer Schwalbenschwanzführung geradegeführt gelagert ist. An dem Schlitten 24 ist obenseitig eine Gewindespindel 25 fest angeordnet, die mittels eines auf dem Ständer 14 fest angeordneten Getriebemotors 26 vertikal auf- und abwärts bewegbar ist. Zu diesem Zweck ist in dem Getriebemotor 26 eine nicht dargestellte Gewindemutter drehbar gelagert und durch den Motor dieses Getriebemotors 26 in beiden Drehrichtungen antreibbar. Die Gewindespindel 25 durchdringt diese Gewindemutter formschlüssig.

An dem Schlitten 24 ist ein horizontaler Ausleger oder Arm 27 fest angeordnet, an dessen vordere Stirnseite ein Getriebemotor 29 mit vertikaler Drehachse fest angeordnet ist. Auf der Ausgangswelle dieses Getriebemotors 29 ist eine Einspannvorrichtung 30 fest angeordnet, die dem Einspannen einer Welle 31 eines als Propeller ausgebildeten Rührorganes 32 dient. Diese Einspannvorrichtung, welche bspw. eine Spannzange aufweist, kann durch ein manuell betätigbares Bedienungsglied, hier einen Ring, geöffnet und geschlossen werden.

In dem Drehtisch 15 ist ein vertikales Durchgangsloch 33′ angeordnet, das in der in Fig. 1 und 3 dargestellten Grundstellung des Drehtisches 15 mit der Drehachse der Einspannvorrichtung 30 fluchtet und es ermöglicht, die Welle 31 zusammen mit dem an ihr fest angeordneten Propeller 32 durch eine Öffnung einer Druckplatte 33 hindurch in die Einspannvorrichtung 30 einzusetzen und wieder herauszunehmen, so daß diese Welle 31 mit dem Rührorgan 32 leicht von Hand auswechselbar ist. Wie weiter unten noch erklärt, kann diese Welle 31 mit dem Rührorgan 32 vorzugsweise ein steril verpackter Einweg-Artikel sein, der nur für eine jeweils einmalige Herstellung eines Knochen zementes gebraucht und dann als Abfall beseitigt wird. Beim Einsetzen der Welle 31 mit Propeller 32 befindet sich die vom Schlitten 24 in weiter unten noch näher beschriebener Weise getragene Druckplatte 33, die man auch als Druckteil bezeichnen kann, in ihrer obersten oder jedenfalls in einer solchen Höhenstellung, daß die Welle 31 mit Propeller 32, wenn sie in das Loch 33′ des Drehtisches eingesetzt ist, von unten her durch das Loch in der Druckplatte 33 eingeführt und dann weiter nach oben bis in die Einspannvorrichtung 30 eingeschoben werden kann. Die Einspannvorrichtung wird dann geschlossen und hält damit die Welle 31 form- und kraftschlüssig fest. Die Abnahme der Welle 31 mit Propeller 32 erfolgt nach Lösen der Einspannvorrichtung 30 auf umgekehrtem Wege.

Die Funktion der Druckplatte 33 wird weiter unten noch näher erklärt. Sie ist auf zwei vertikalen Führungsstangen 34 vertikal geradegeführt, auf denen sie ständig belastende Druckfedern 35 angeordnet sind. Die Druckfedern 35 drücken sie auf an den Führungsstangen 34 untenseitig befestigte Anschläge, wie 36′, von denen sie abgehoben wird, wenn diese Druckplatte 33 einen an ihr untenseitig zur Anlage kommenden Deckel 36 mit einer an ihm untenseitig angeordneten elastischen Dichtung 37 auf den oberen Rand der jeweiligen Kartusche 20 drückt.

Die beiden Führungsstangen 34 sind obenseitig an horizontalen Armen 39 befestigt, die fest an dem Gehäuse des Getriebemotors 29 befestigt sind und an denen sich die Druckfedern 35 obenseitig abstützen.

Der Deckel 36, der vorzugsweise ebenfalls ein steril verpackter Einweg-Artikel ist, da er nur dem Verschließen der Kartusche 20 während der Vorbereitung eines einzigen Knochenzementes dient und deshalb nach einmaligem Gebrauch zusammen mit der Kartusche als Abfall beseitigt werden kann, besteht im wesentlichen aus einem Kunststoffkörper, der ein mittiges Durchgangsloch aufweist, welches von der Welle 31, durchdrungen ist, wobei die Welle 31 zwei O-Ringe 39′ des Deckels 36 durchdringt, die der luftdichten Abdichtung des von der Welle 31 durchdrungenen Loches dienen, ohne die axiale Verschiebbarkeit der Welle 31 relativ zu dem Deckel 36 zu behindern. Dieser Deckel 36 wird auf die Welle 31 aufgeschoben und durch die O-Ringe 39′ reibungsschlüssig gehalten, bevor diese in die Einspannvorrichtung 30, wie oben beschrieben, eingespannt wird.

Dieser Deckel 36 weist einen vertikalen Luftkanal 40 auf und wird beim Einsetzen der Welle 31 in die Einspannvorrichtung 30 in eine solche Winkelstellung zur Druckplatte 33 gedreht, daß dieser Luftkanal 40, wenn diese Druckplatte 33 an den Deckel 36 angedrückt wird, mit einem abgewinkelten Luftkanal 41 der Druckplatte 33 kommuniziert, also dann, wenn die Druckplatte 33 an der Oberseite des Deckels 36 zwecks Andrücken des Deckels 36 an den oberen Rand der Kartusche 20 anliegt. Eine in den Deckel 36 eingelassen Ringdichtung 42 dichtet dabei die Luftkanäle 40 und 41 an ihrer Verbindungsstelle nach außen ab.

An den Luftkanal 41 in der Druckplatte 33 ist eine flexible Luftleitung 43 angeschlossen, die zu einem in Fig. 1 gestrichelt angedeuteten, im Kasten 13 untergebrachten 3-Wege-Ventil 44 führt, dessen beiden anderen Anschlüsse an Luftleitungen 45 und 46 angeschlossen sind.

In die Luftleitung 45 sind eine als Unterdruckquelle dienende Saugpumpe 47 und ein Aktivkohlefilter 49 und in die Luftleitung 46 ein Sterilfilter 50 zwischengeschaltet. Dieses 3-Wege-Ventil 44 ist mittels eines Stellmotors 51 umschaltbar.

Der den Drehtisch 15 drehende Motor 17 kann vorzugsweise ein pneumatischer oder hydraulischer Arbeitszylinder sein, jedoch auch andere Bauart haben, bspw. ein Elektromotor sein.

In dem Kasten 13 ist eine Programmsteuer- und/oder Prozeßsteuervorrichtung 52 angeordnet, die der Programmsteuerung und/oder der Prozeßsteuerung der Mischphase und der Ruhephase bei der Herstellung des jeweiligen Knochenzementes dient und/oder der Durchführung mindestens eines prozeßgesteuerten Schrittes bei der Vorbereitung des jeweiligen Knochenzementes dienen kann.

Diese Vorrichtung 52 wird im weiteren zur Vereinfachung als "Steuervorrichtung" bezeichnet.

An diese Steuervorrichtung 52 ist ein Temperaturfühler 58 angeschlossen, der die Raumtemperatur des Opera tionssaales fühlt, in welchem die Maschine 10 aufgestellt ist.

Die Steuervorrichtung 52 dient der selbsttätigen kompletten Steuerung der Vorbereitung des jeweiligen Knochenzementes in einer Kartusche 20.

An die Steuervorrichtung 52 ist ein Lesegerät 53 angeschlossen, das auf dem Drehtisch 15 angeordnet ist und auf dem Schlitten 19 angeordnete, codierte Daten bildende Markierungen oder dgl. durch einen am Drehtisch angeordneten Längsschlitz oder Schlitz 54 hindurch lesen kann. Es handelt sich hier um Daten, die den Typ des herzustellenden, d.h. vorzubereitenden Knochenzementes und dessen Menge angeben und das Lesegerät 53 liest diese Daten vorzugsweise berührungslos und signalisiert sie einem Rechner 55 der Steuervorrichtung 52. An diesen Rechner 55 ist ein Speicher 56 angeschlossen. In diesem Speicher 56 sind Daten in Form eines Datenfeldes oder eines Kennlinienfeldes gespeichert, wie sie vom Rechner 55 benötigt werden, um aus ihnen die für die selbsttätige Steuerung der der Vorbereitung des jeweiligen Knochenzementes dienenden Steuerschritte für die Mischphase und die Ruhephase zu berechnen und dann diese beiden Phasen entsprechend selbsttätig und für den betreffenden Knochenzement und seine jeweils vorgesehene Menge zu steuern, um so den jeweiligen Knochenzement reproduzierbar und selbsttätig vorzubereiten.

Für jeden auf dieser Maschine 10 vorzubereitenden Knochenzementtyp können dabei im Speicher 56 Daten über die zu berechnenden Steuerschritte für unterschiedliche, vom Raumtemperaturfühler 58 gefühlte Raumtemperaturen gespeichert sein, wie sie in dem Operationssaal je nach Operation geregelt werden.

In diesen Speicher 56 können auch nachträglich Daten für ursprünglich nicht vorgesehene Knochenzementtypen gespeichert werden und frühere Daten für Knochenzementtypen, die nicht mehr benutzt werden, können wieder gelöscht werden.

Beispielsweise können die Daten für einen bestimmten Knochenzementtyp in Form von Kennlinien oder Datenreihen gespeichert sein, wobei der Parameter der Kennlinien dieses Kennlinienfeldes bzw. die Datenreihen eines Datenkennfeldes die vom Raumtemperaturfühler 58 gefühlte Raumtemperatur im Operationssaal oder die von einem Temperaturfühler 72' (Fig .5) gefühlte Temperatur der jeweiligen Kartusche oder gefühlte Innentemperatur der Kartusche 20 ist. Die betreffende Kennlinie oder Datenreihe gibt dann in Abhängigkeit der Menge des vorzubereitenden Knochenzementes für den betreffenden Knochenzement dem Rechner 55 die Daten an, aus denen er dann die Steuerschritte für die Mischphase bzw. die Ruhephase berechnet, wobei auch während der Steuerung noch prozeßabhängige Daten, bspw. die jeweilige Temperatur der Kartusche, dem Rechner 55 zur Korrektur der im Ablauf befindlichen Steuerschritte signalisiert werden können.

Solche Datenreihen können experimentell für jeden Knochenzementtyp erarbeitet werden. Nachfolgendes Beispiel einer solchen Datenreihe eines Knochenzementes aus der Gruppe der sogenannten "Standard Viscosity" Knochenzemente soll dies noch näher erläutern.

| Raumtemperatur | Komponententemperatur | Rührzeit | Ruhephase bis | |
|---|---|---|---|---|
| °C | °C | Sek. | Min | Sek. |
| 17 | 4 | 30 | 4 | 30 |
| 20 | 4 | 30 | 4 | 00 |
| 23 | 4 | 30 | 3 | 30 |
| 17 | 8 | 25 | 4 | 00 |
| 20 | 8 | 25 | 3 | 30 |
| 23 | 8 | 25 | 3 | 00 |

Es sei hier ferner noch ausgeführt, daß bei den sogenannten "low viscosity" Knochenzementen die Komponenten meist nicht oder nur bei sehr hohen Raumtemperaturen gekühlt werden sollten, damit die Ruhephase nicht unnötig lang wird.

Einige Arbeitsweisen dieser Maschine 10 seien nachfolgend beschrieben.

Für eine vorzunehmende Operation wird festgelegt, welcher Knochenzementtyp verwendet und wie groß die Menge des herzustellenden Knochenzementes ist. Der als Datenträger dienende Schlitten 19 wird mit den Daten über den Knochenzementtyp und der Menge des herzustellenden Knochenzementes versehen und in ihn die zunächst noch leere Kartusche 20 eingesetzt. Der Schlitten 19 mit Kartusche 20 wird dann in die Geradführung 21 des Drehtisches 15 in seine vorgesehene Stellung bis zum Anschlag eingeschoben. Die Welle 31 mit dem Propeller 32 und dem auf sie aufgeschobenen Deckel 36 wird in die Einspannvorrichtung 30 eingesetzt. Die Komponenten des herzustellenden Knochenzementes befinden sich in zwei Vorratsgefäßen 59, 60, die auf eine Haltevorrichtung 61 aufgesteckt werden, die am Ständer 14 angeordnet ist. Das Vorratsgefäß 59 enthält flüssiges Monomer ggfs. mit Zusätzen von Härter oder dergl. und das Vorratsgefäß 60 enthält die pulverförmige Komponente des herzustellenden Knochenzementes. Es kann ggfs. auch vorgesehen sein, in die Kartusche 20 in konventioneller Weise den vorgegebenen Inhalt einer Monomerampulle und eines Polymerpulverbeutels von Hand einzufüllen.

Diese Vorratsgefäße 59, 60 enthalten in diesem Ausführungsbeispiel diese Komponenten im Überschuß und es ist deshalb zur genauen Dosierung der für die herzustellende Menge des Knochenzementes benötigten Mengen dieser Komponenten an dieser Haltevorrichtung 61 je eine von Hand einstellbare Dosiervorrichtung 62, 63 angeordnet. An diese Dosiervorrichtungen 62, 63 werden die Vorratsbehälter 59, 60 über kurze Leitungen angeschlossen, und wobei auch nicht dargestellte Entlüftungsleitungen mit angeschlossen sein können, um problemlose Entnahme der betreffenden Mengen an Komponenten aus diesen Vorratsbehältern 59, 60 zu ermöglichen.

An die Ausgänge der beiden Dosiervorrichtungen 62, 63 wird je eine flexible Leitung 64, 65 angeschlossen und in die offene Kartusche 20 eingeführt, wie es in Fig. 1 strichpunktiert dargestellt ist. Es kann nun vorgesehen sein, daß die Dosiervorrichtungen 62, 63 manuell aktiviert werden, oder es kann auch vorgesehen sein, daß sie von der Steuervorrichtung 52 aktiviert werden. Im Gefolge ihrer Aktivierung entnehmen sie aus den Vorratsbehältern die eingestellten Mengen an den beiden Komponenten, welche so durch die Leitungen 64, 65 hindurch in die Kartusche 20 eingeführt werden.

Nachdem die beiden Komponenten in die Kartusche in den vorbestimmten Mengen eingefüllt sind, können die Leitungen 64 und 65 aus ihr herausgezogen werden oder sie ziehen sich von selbst beim nunmehr erfolgenden Einschalten des Drehens des Drehtisches 15 aus der in Fig. 1 dargestellten, der Grundstellung entsprechenden Position in seine in diesem Ausführungsbeispiel um ca. 90° winkelversetzte Arbeitsposition, die in Fig. 2 dargestellt ist. In dieser Arbeitsstellung findet das Verrühren der in die Kartusche 20 eingefüllten Komponenten und vorzugsweise auch die anschließende Ruhephase statt. Das Einschalten dieser Drehbewegung, die durch einen nicht dargestellten Endschalter beendet werden kann, kann manuell oder durch die Steuervorrichtung 52 erfolgen. In dieser Arbeitsposition befindet sich unmittelbar gegenüber der Geradführung 21 des Drehtisches 15 außenseitig am Kasten 13 ein als Sperre dienendes, bspw. metallisches Schild 66, das es unmöglich macht, den Schlitten 19 mit der Kartusche 20 aus der Geradführung 21 herauszunehmen, wodurch sichergestellt wird, daß während des Vermischens der Komponenten und ggfs. auch während der Ruhephase die Kartusche 20 nicht aus dem Drehtisch 15 versehentlich entnommen werden kann.

Sobald der Drehtisch 15 in seiner vorgesehenen Arbeitsstellung angekommen ist, kann der Arm 27 zusammen mit den von ihm getragenen Teilen mittels des Motors 26 abgesenkt werden bis er in eine vorbestimmte Stellung gelangt ist, in der der Propeller 32 sich innerhalb der Kartusche 20 befindet und der Deckel 36 an den oberen Rand der Kartusche 20 deren Innenraum nach außen luftdicht abdichtend angedrückt ist. Dies wird der Steuervorrichtung 52 bspw. mittels eines Endschalters signalisiert und diese aktiviert jetzt spätestens das Lesegerät 53 zum Lesen der auf dem Schlitten 19 enthaltenen Angaben über die Menge und den Typ des betreffenden Knochenzementes. Das Lesegerät 53 signalisiert dies dem Rechner 55, der nunmehr vorzugsweise im Dialog mit dem Speicher 56 die für die Steuerung des Vermischens der in der Kartusche 20 befindlichen Komponenten des Knochenzementes und die für die anschließende Ruhephase erforderlichen Daten in der vorgesehenen Reihenfolge berechnet, wobei diese Daten den vom Raumtemperaturfühler 58 gemeldeten Wert der Raumtemperatur für den betreffenden Knochenzementtyp und dessen Menge berücksichtigen.

Gleichgültig, ob die Steuervorrichtung 52 bereits das Einfahren des Propellers 32 in die Kartusche steuert oder ob das Absenken des Armes 27 manuell ausgelöst wird, kann während des Einfahrens des Propellers 32 in die Kartusche 20 vorgesehen sein, daß sich die Welle 31 und damit der als Rührorgan dienende Propeller 32 bereits dreht oder es kann auch zugewartet werden, bis der Propeller 32, den man auch als Flügelrad bezeichnen kann, in eine vorgesehene Stellung innerhalb der Kartusche 20 angelangt ist, bei der er gegebenenfalls schon in die in der Kartusche 20 befindlichen Komponenten eingetaucht sein kann, bzw. bis die Steuervorrichtung 52 die Steuerung des Getriebemotors 29 übernimmt. Wenn sich der Propeller 32 beim Einführen in die Kartusche bzw. in die in ihr befindlichen Komponenten schon dreht, kann dies vorzugsweise mit einer ersten niedrigen Drehzahl stattfinden. Es kann dann zu einem programmierten späteren Zeitpunkt eine höhere Drehzahl des Propellers 32 eingeschaltet werden oder es wird diese höhere Drehzahl ausschließlich eingeschaltet. Auch andere Möglichkeiten bestehen, bspw. programmierte oder prozeßgesteuerte stetige oder mehrfach stufenweise Verstellung der Drehzahl des Propellers 32 während des Vermischens.

Der Rotationsradius des Propellers 32 ist nur geringfügig kleiner als der Innendurchmesser der Kartusche 20, was möglich ist, da die Drehachse der Welle 31 mit der Längsachse der Kartusche 20 in der Arbeitsstellung des Drehtisches 15 fluchtet. Auch ist der Propeller 32 untenseitig ungefähr eben und kann bis fast auf den Boden der Kartusche 20 abgesenkt werden.

Nunmehr findet das Mischen der in der Kartusche 20 befindlichen Komponenten miteinander durch das Rühr organ 32 statt. Dieses wird in Abhängigkeit der in der Kartusche 20 befindlichen Menge an Knochenzementkomponenten, d. h. der Füllhöhe in der Kartusche nunmehr während der Mischphase zum Mischen fortlaufend auf und ab bewegt, sei es während einer programmierten Zeitspanne oder während einer vorbestimmten Anzahl von Auf- und Abwärtshüben oder prozeßgesteuert mittels des reversierbaren Getriebemotores 26, der hierzu entsprechend von der Steuervorrichtung 52 angesteuert wird. Die Hubhöhe ist dabei so eingestellt, daß der Propeller 32 ständig in die Knochenzementmasse in der Kartusche 20 eingetaucht bleibt und der Aufwärtshub vorzugsweise so programmiert ist, daß er bis dicht unter die Füllhöhe dieser Masse in der Kartusche 20 führt und der Abwärtshub führt jeweils zweckmässig (wegen besonders gleichmässigen Vermischens) bis dicht über den Boden 18 der Kartusche 20. Aus den im Speicher 56 gespeicherten Daten rechnet der Rechner 55 nicht nur die Hubhöhe des Propellers 32 für dessen Hubbewegungen aus, sondern auch unter Berücksichtigung der Raumtemperatur und der Füllmenge die für das innige Vermischen der beiden Komponenten zum Knochenzement erforderliche Zeitdauer oder die Anzahl der Hübe des Propellers 32 bis zum Ende dieser Mischphase.

Sobald der Deckel 36 durch die Druckplatte 33 an die Kartusche 20 angedrückt worden ist, wird die die Unterdruckquelle bildende Saugpumpe 47 eingeschaltet und bewirkt einen vorbestimmten Unterdruck der Kartusche während des Rührens oder zumindest zeitweise während des Rührens. Dieser Unterdruck kann bspw. 300 bis 700 mm Hg, vorzugsweise 400 bis 600 mm Hg, betragen. Es ist also keine völlige Luftleere der Kartusche 20 erforderlich, sondern in ihr kann ein vorbestimmter Unterdruck hergestellt werden, der erheblich niedriger als der Atmosphärendruck im Operationssaal ist. Diese Saugpumpe 47 dient dazu, um den Knochenzement möglichst luftfrei zu machen, insbesondere Lufteinschlüsse zu verhindern. Die Unterdruckpumpe 47 oder Vakuumpumpe kann auch während der anschließenden Ruhephase zumindest zeitweise eingeschaltet bleiben oder während ihr abgeschaltet sein. Die Steuervorrichtung 52 schaltet den Motor 26 am Ende dieser Mischphase vorzugsweise in einer vorbestimmten Hubstellung des Propellers 32 diesen hierdurch anhaltend aus. Und zwar kann hier zweckmäßig vorgesehen sein, daß der Propeller 32 vor seinem Anhalten aus dem Knochenzement herausgeführt und an ihm anhaftenden Knochenzement mit hoher Drehzahl noch innerhalb der Kartusche 20 abschleudert und erst dann stillgesetzt wird.

Nunmehr beginnt die Ruhephase, während welcher die Polymerisation des Knochenzementes langsam fortschreitet. Der Rechner 55 berechnet aus den in ihn eingegebenen Daten die Zeitdauer dieser Ruhephase oder diese wird prozeßabhängig beendet, bspw. wenn die durch Polymerisieren des Knochenzementes ansteigende Temperatur einen vorbestimmten Differenzwert zur Raumtemperatur erreicht. Wenn die Ruhephase beendet ist oder schon vorher, steuert die Steuervorrichtung das Hochfahren des Schlittens 24 nach oben in seine in Fig. 1 dargestellte Stellung, in der der Deckel 36 wieder von der Kartusche 20 abgenommen und der Pro peller 32 aus der Kartusche 20 nach oben herausgefahren ist. Am Ende der Ruhephase, bzw. wenn erst am Ende der Ruhephase der Propeller 32 aus der Kartusche 20 herausgefahren wird, befiehlt nach diesem Herausfahren die Steuervorrichtung 52 den Drehtisch 15 aus der Arbeitsposition (Fig. 2) in die Grundstellung nach Fig. 1 zu überführen, indem der Motor 17 entsprechend eingeschaltet wird. Diese Drehbewegung kann bspw. mittels eines Endschalters beendet werden. Sobald der Drehtisch 15 seine in Fig. 1 dargestellte Grundstellung wieder eingenommen hat, kann dies optisch oder akustisch signalisiert werden, und eine Bedienungsperson, bspw. eine Schwester, nimmt nunmehr den Schlitten 19 mit der Kartusche 20 aus der Geradführung 21 heraus. Auf diese Kartusche 20 wird dann ein einen Spritzkanal aufweisender Schraubdeckel aufgeschraubt und der Operateur nimmt nunmehr das Einfüllen des Knochenzementes in die betreffende Knochenhöhlung des Patienten vor, was durch Verschieben des beweglichen Bodens dieser Kartusche 20, die einen Kolben bildet, bekannterweise erfolgt.

An der Maschine 10 ist noch ein Display 68 angeordnet, auf dem für das Bedienungspersonal wichtige oder benötigte Daten angezeigt werden können, bspw. der Typ des Knochenzementes und dessen Füllmenge. Desgleichen kann der Fortgang der Mischphase und der Ruhephase angezeigt werden. Auch kann vorgesehen sein, daß auf diesem Display 68 nach Beendigung der Ruhephase die dem Operateur zur Verfügung stehende Verarbeitungszeit des Knochenzementes signalisiert wird, bspw. durch Rückwärtszählung der Zeitdauer, um jeweils anzuzeigen, welche Zeitspanne ihm bis zur Beendigung des Einsetzens der Prothese in die richtige Stellung zur Verfügung steht. Für den betreffenden Knochenzementtyp und seine Menge kann auch die Aushärtungszeit angezeigt werden, bspw. ebenfalls durch Rückwärtszählung. Auch diese Anzeigen können von der Steuervorrichtung 52 bewirkt und gesteuert werden.

Es sei noch erwähnt, daß der durch die Saugpumpe 47 in der Kartusche 20 zu erzeugende Unterdruck auch gesteuert oder geregelt werden kann, bspw. dadurch, indem diese Pumpe 47 drehzahlsteuerbar ist oder ihr Eingang nicht nur an das 3-Wege-Ventil angeschlossen ist, sondern über ein Steuerventil 93 (Fig .1) noch Falschluft bspw. aus dem Innenraum des Kastens 13, gesteuert durch eine Steuerung oder durch einen Unterdruckregler, die bzw. der der Teil der Steuervorrichtung 52 sein kann, ansaugen kann. Im Falle der Regelung bewirkt das Steuerventil 93, daß Falschluft von der Pumpe 47 in solchem Maße mit angesaugt wird, daß ein eingestellter konstanter Unterdruck in der Kartusche 20 erzeugt wird, indem es durch den Unterdruckregler in Abhängigkeit der die Regelabweichung bildenden Differenz zwischen einem eingestellten Sollwert des Unterdruckes und dem von einem Druckfühler 94 in der Leitung 43 gefühlten Unterdruck so durch einen Stellmotor verstellt wird, dass diese Regelabweichung verkleinert wird. Im Falle der Steuerung des Unterdruckes wird das Steuerventil 93 gemäß dem jeweils gewünschten Unterdruck eingestellt.

Der Beginn der Mischphase kann unterschiedlich vorge sehen sein. Im einfachsten Fall führt die Bedienungsperson der Maschine 10 das Einleiten der Komponenten des Knochenzementes in die Kartusche durch, indem sie von Hand die Dosiervorrichtungen 62, 63 einschaltet und nach Beendigung des Einfüllens der Komponenten in die Kartusche 20 einen Schalter betätigt, der dem Drehen des Drehtisches 15 aus seiner Grundstellung in die Arbeitsstellung auslöst. Der Endschalter, der das Erreichen dieser Arbeitsstellung des Drehtisches 15 signalisiert, kann dann die Steuervorrichtung 52 für das Auslösen des weiteren selbsttätigen Laufes der nunmehr beginnenden Mischphase und der anschließenden Ruhephase ansteuern, wobei die Steuervorrichtung dann zuerst Absenken der Druckplatte 33 zum Andrücken des Deckels 36 auf die Kartusche und entsprechend das Einführen des Propellers 32 in die Kartusche 20 steuert. Oder es kann auch vorgesehen sein, daß auch dieser Vorgang des Absenkens dieser Druckplatte 33 beim Drücken des Deckels 36 an die Kartusche 20 und Einführen des Propellers 32 in sie noch von einer Bedienungsperson von Hand ausgelöst wird und erst dann, wenn die Druckplatte 33 den Deckel 36 an die Kartusche angedrückt hat, ein Endschalter oder die Bedienungsperson die Steuervorrichtung 52 zur nunmehr erst beginnenden selbsttätigen Durchführung der Mischphase und der Ruhephase auslöst.

Besonders vorteilhaft ist, wenn der selbsttätige Ablauf der Mischphase begonnen wird, sobald die Bedienungsperson die Vorratsbehälter 59, 60 auf die Haltevorrichtung 61 aufgesteckt und die Leitungen 64, 65 in die Kartusche 20 in der Grundstellung des Dreh tisches 15 eingeführt hat. Es kann dann vorgesehen sein, daß entweder die Bedienungsperson vorher oder erst jetzt die Dosiervorrichtung 62, 63 von Hand auf die abzumessenden Mengen der beiden Komponenten einstellt oder daß dies von der Steuervorrichtung 52 selbsttätig vorgenommen wird, nachdem ihr vom Lesegerät 53 der Knochenzementtyp und die Füllmenge signalisiert worden ist, in welchem letzteren Falle die Leitungen 64, 65 nicht in der Grundstellung des Drehtisches 15 in die Kartusche 20 eingeführt werden, sondern vor dem Einführen dieser Leitungen 64, 65 wird von Hand oder durch die Steuervorrichtung bereits Drehen des Drehtisches 15 in seine Arbeitsstellung (Fig. 2) befohlen und dann erst werden die Leitungen 64, 65 eingeführt. In diesem Fall kann dann die Steuervorrichtung 52 die erforderlichen Einstellungen der Dosiervorrichtungen 62, 63 und deren Arbeit steuern und anschließend kann sie auch mittels eines geeigneten Mechanismus das Absenken der Druckplatte zusammen mit dem Deckel 36 und dem Propeller 32 auf bzw. in die Kartusche 20 steuern.

Der Beginn der Mischphase kann also unterschiedlich vorgesehen sein. Im Extremfall beginnt die selbsttätige Steuerung der Mischphase durch die Steuervorrichtung 52 bereits vor Einfüllen der in den Vorratsbehälter 59, 60 befindlichen Komponenten in die Kartusche 20, wobei dann alle danach für die Vorbereitung des Knochenzementes erforderlichen Schritte durch die Steuervorrichtung 52 gesteuert werden, wie Einstellung der Dosiervorrichtungen 62, 63, ebenfalls auch Einführen der Leitungen 64, 65 in die Kartusche 20, Einfüllen der Komponenten in die Kartusche 20, Absenken der Druckplatte 33 mit Deckel 36 und Propeller 32 bis auf bzw. in die Kartusche 20, anschließendes Verrühren der Komponenten, die sich in der Kartusche 20 befinden, miteinander, und anschliessende Steuerung der Ruhezeit.

Im anderen Extremfall beginnt die Mischphase, die von der Steuervorrichtung 52 selbsttätig zusammen mit der anschließenden Ruhephase gesteuert wird, erst dann, wenn durch die Bedienungsperson bewirkt, die beiden Komponenten in die Kartusche 20 bereits eingefüllt sind, der Drehtisch 15 in seine Arbeitsstellung gedreht und die jeweilige Kartusche 20 bereits durch den Deckel 36 abgeschlossen und der Propeller 32 bereits in die in der Kartusche befindlichen Komponenten oder jedenfalls in die Kartusche 20 bereits eingeführt ist.

Der Beginn der selbsttätigen Steuerung der Mischphase kann auch zwischen diesen beiden Extremen vorgesehen sein, bspw. mit dem Erreichen der Arbeitsstellung des Drehtisches 15 oder mit Beendigung des Einfüllens der Komponenten in die Kartusche 20 begonnen werden. Auch sonstiger anderer Beginn der selbsttätigen Steuerung der Mischphase durch die Steuervorrichtung 52 kann zwischen den beiden

EP 0 402 735 A1

genannten Extremen vorgesehen sein.

Im Sinne der Erfindung beginnt also die Mischphase zu dem Zeitpunkt, ab dem die Steuervorrichtung 52 tätig wird.

Gemäß vorstehender Beschreibung einiger möglicher Arbeitsweisen der Steuervorrichtung 52 kann diese als reine Programmsteuervorrichtung arbeiten. Oder es kann jedoch auch zusätzlich oder, wie noch weiter unten erläutert wird, auch Prozeßsteuerung stattfinden.Bspw. kann die Temperatur der Kartusche 20 während des Mischens und gegebenenfalls auch während der Ruhephase durch einen Temperaturfühler gefühlt und aus dem gefühlten Temperaturanstieg, der während des Fortschreitens der Polymerisation auftritt, auf den Polymerisationszustand des Knochenzementes, insbesondere auf seine Viskosität geschlossen und mindestens ein Steuerschritt in Abhängigkeit dieses Polymerisationszustandes gesteuert werden. Bspw. kann die Mischphase und/oder die Ruhephase in Abhängigkeit dieses Polymerisationszustandes und damit prozeßabhängig beendet werden.

Anstatt des Drehtisches 15 kann auch ein linear oder sonstwie bewegbarer Tisch vorgesehen sein, der zwischen einer Grundstellung, in der die Kartusche 20 mit Schlitten 19 in eine entsprechende Geradführung 21 eingeschoben wird und einer Arbeitsstellung, in der die Entnahme des Schlittens 19 mit Kartusche 20 aus dieser Geradführung gesperrt ist, lageverstellbar ist.

Auch ist es möglich, die Geradführung 21 für den Schlitten 19 am Kasten 13 u.a. an oder in dessen Oberseite unbeweglich anzuordnen und die Entnahme des Schlittens 19 mit Kartusche 20 während der Arbeit des Rührorganes 32 und ggfs. auch während der Ruhephase auf andere Weise zu sperren, bspw. mittels eines selbsttätig bewegbaren Riegels oder es kann auch vorgesehen sein, auf eine solche Sperre zu verzichten und es der Genauigkeit des Bedienungspersonals zu überlassen, daß keine versehentliche vorzeitige Entnahme der Kartusche 20 aus der Maschine stattfindet. Oder diese vorzeitige Entnahme wird dadurch gesperrt, indem die Druckplatte 33 den Deckel 36 bis zum Ende der Ruhephase auf die Kartusche drückt. Die Ruhephase ist in diesem Fall dann beendet, sobald die Druckplatte 33 zusammen mit dem Deckel 36 und dem Propeller 32 von der Kartusche weggeführt wird.

Es kann in manchen Fällen auch vorgesehen sein, daß die Ruhephase oder ein Teil der Ruhephase bei bereits abgenommenem Deckel 36 stattfindet, sofern die aus der Kartusche dabei möglicherweise entweichenden Monomerdämpfe nicht stören oder abgesaugt werden.

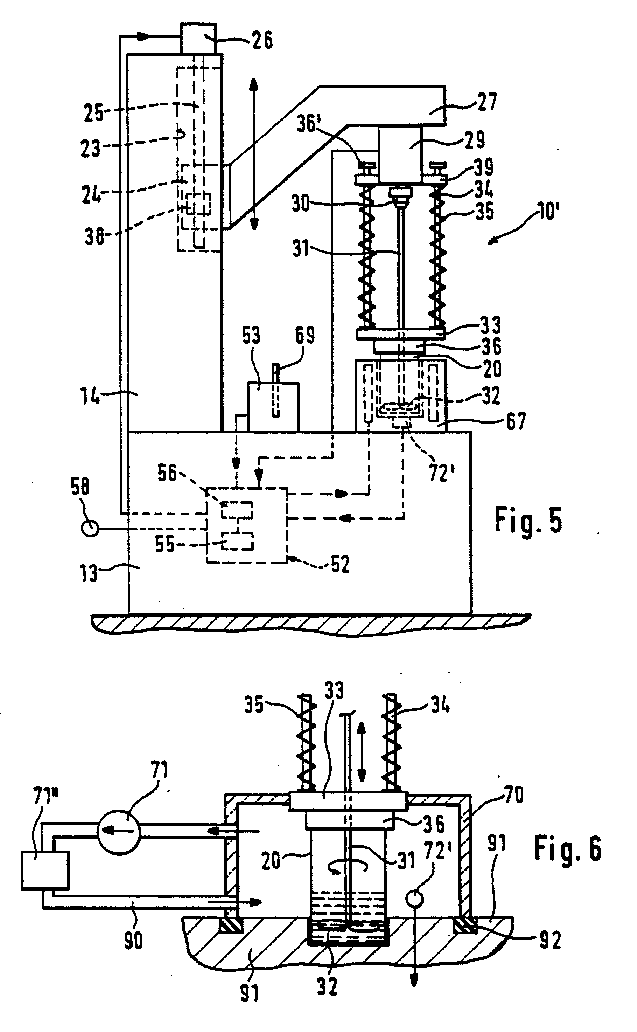

Bei der in Fig.5 dargestellten Maschine 10′ sind Teile, die Teilen der Maschine 10 nach den Fig.1 bis 4 entsprechen oder entsprechen können oder gleiche Aufgaben haben, mit gleichen Bezugszeichen bezeichnet und bedürfen deshalb keiner näheren Erläuterung.

Diese ebenfalls eine Vorrichtung zum Vorbereiten von Knochenzement für das nachfolgende Einführen in eine der Aufnahme einer Prothese dienende Knochenhöhle eines Knochens darstellende Maschine 10′ nach Fig. 5 unterscheidet sich von der nach Fig. 1 bis 4 im wesentlichen dadurch, daß die jeweilige Kartusche 20 am Kasten 13 des Gestelles 11 unbeweglich angeordnet wird. Sie ist hier in eine Ausnehmung einer Temperier vorrichtung 67 über diese etwas überstehend formschlüssig eingesetzt und in sie bereits der Propeller 32 eingefahren dargestellt.

Das Lesegerät 53 dient hier nicht dem Lesen von auf einem die jeweilige Kartusche 20 tragenden Schlitten aufgebrachten Daten, sondern diese Daten befinden sich auf einem gesonderten, bspw. als Karte ausgebildeten Datenträger 69.

Die Temperiervorrichtung 67 dient dem Kühlen und/oder Erwärmen der Kartusche 20, damit der in ihr jeweils befindliche Knochenzement in seiner Polymerisationsgeschwindigkeit in vorbestimmter Weise beeinflußt wird, die besonders genaue Steuerung der Ruhephase bzw. besonders gute Steuerung des Vermischens ermöglicht. Auch läßt sich mittels dieser Temperiervorrichtung 67 die Verarbeitungszeit, die dem Operateur nach Beendigung der Ruhephase zur Verfügung steht, beeinflussen, wenn der Knochenzement am Ende der Ruhephase eine vorbestimmte, durch die Temperiervorrichtung 67 bewirkte erhöhte oder erniedrigte Temperatur hat.

In Fig. 5 ist die Maschine 10′ in einem Arbeitszustand dargestellt, in der sich der Propeller 32 bereits in der betreffenden Kartusche 20 befindet. Er wurde vorher, die bei der Maschine nach den Fig. 1 bis 4, in die Kartusche 20 aus einer im Abstand oberhalb der Kartusche befindlichen Stellung unter Mitbewegen des Deckels 36 durch Abwärtsbewegen der ebenfalls federnd abgestützten Druckplatte 33 eingefahren, nachdem vorangehend die Komponenten des betreffenden Knochenzementes in die Kartusche 20 eingefüllt wurden.

In diesem Ausführungsbeispiel sind die Führungsstangen 34 an der Druckplatte 33 fest angeordnet und durch die am Getriebemotor 29 befestigten Arme 39, und zwar durch Führungslöcher dieser Arme 39 geradegeführt und weisen an ihren oberen Enden die Abwärtsbewegung der Druckplatte 33 relativ zum Ausleger 27 begrenzende Anschläge 36′ obenseitig auf.

Die Gewindespindel 25 ist hier mit der Ausgangswelle des Getriebemotors 26 fest verbunden und kämmt mit einer von ihr durchdrungenen, am Schlitten 24 fest angeordneten Gewindemutter 38.

In Fig. 6 ist eine Abwandlung der Temperiervorrichtung 67 der Fig. 5 dargestellt. Sie weist eine an der Druckplatte 33 befestigte Haube 70 auf, die sich in ihrer normalen Stellung weit oberhalb der jeweils in einer Ausnehmung des unbeweglichen Tisches 91 des Kastens 13 befindlichen Kartusche 20 befindet. Wenn auf den Tisch 91 eine Kartusche aufgesetzt ist und in sie die jeweiligen Komponenten des herzustellenden Knochenzementes eingebracht sind und die Welle 31 mit dem Flügelrad 32 in die Einspannvorrichtung 30 eingespannt ist, dann wird die Haube 70 durch Absenken des Armes 27 soweit zusammen mit der Druckplatte 33 nach unten bewegt, bis sie auf einer in den Tisch 91 eingelassenen Ringdichtung 92 aufsitzt. In dieser Stellung der Haube 70 drückt die federnd abgestützte Druckplatte 33 den auf der Welle 31 befindlichen Deckel 36 an den oberen Rand der betreffenden Kartusche 20 an. Diese Haube 70 bildet so einen nach außen abgedichteten Raum, innerhalb welchem sich die Kartusche 20 während des Vermischens der in sie vorangehend eingefüllten Komponenten des Knochenzementes und vorzugsweise auch während der Ruhephase des Knochenzementes befindet. Der Innenraum der Haube 70 ist an eine externe Pumpe 71 angeschlossen, die in eine in die Haube 70 zurückführende Umluftleitung 90 zwischengeschaltet ist.

Die von dieser Pumpe 71 angesaugte Luft wird durch eine ebenfalls in die Umluftleitung 90 zwischengeschaltete Heizvorrichtung und/oder Kühlvorrichtung 71" hindurchgeleitet und wird in den Innenraum der Haube 70 als erwärmte oder gekühlte Umluft zurückgeleitet, wodurch die jeweilige Kartusche 20 und damit die in ihr befindliche Knochenzementmasse in vorbestimmter, durch die Steuervorrichtung 52 steuerbarer und vorzugsweise auch durch einen Regler regelbarer Weise temperiert wird. Um diese Temperatur zu regeln, kann ein Temperaturfühler 72' innerhalb der Haube 7o angeordnet sein, der der Istwertfühler des Istwertes der durch einen nicht dargestellten Regler zu regelnden Innentemperatur der Haube 70 und damit der Temperatur der Kartusche 20 und des in ihr befindlichen Knochenzementes bzw. dessen Komponenten ist. Dieser Regler kann ein Teil der Steuervorrichtung 52 sein und ihr einstellbarer Sollwert kann durch den Rechner 55 berechnet oder auf sonstige Weise eingegeben werden, bspw. oft zweckmässig auch manuell.

Auch bei der Maschine nach Fig. 5 ist in dem Innenraum der Temperiervorrichtung 67 ein Temperaturfühler 72' angeordnet, der die Temperatur der Kartusche 20 und damit die Temperatur der in ihr jeweils befindlichen Knochenzementmasse fühlt und sie einem nicht dargestellten Regler zur Regelung dieser Innentemperatur signalisiert.

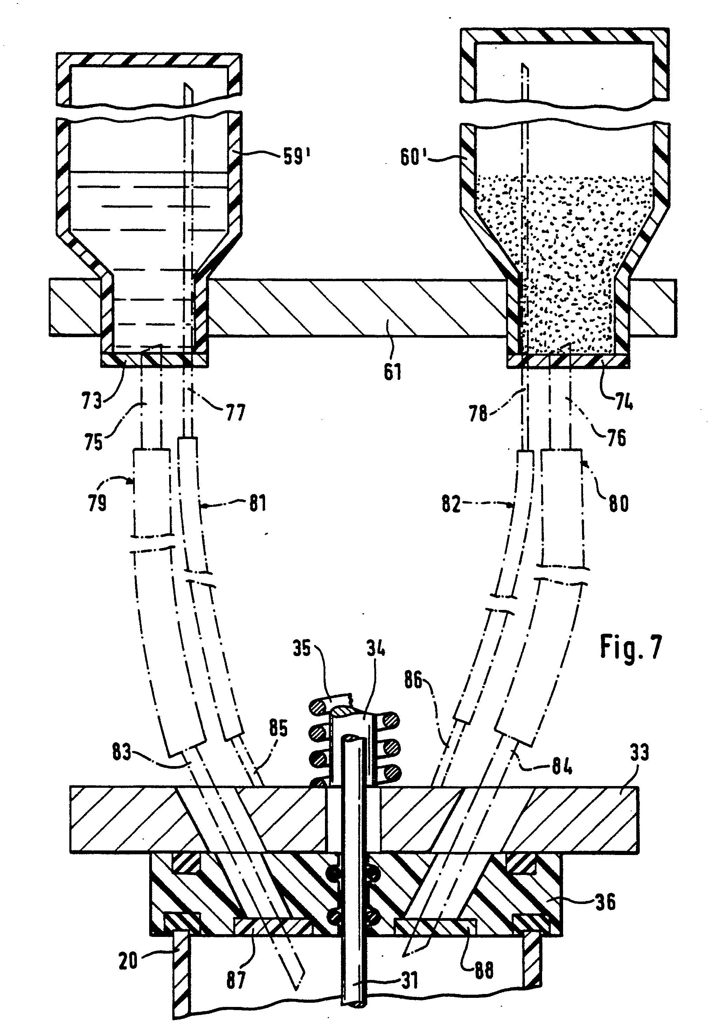

Die Komponenten des jeweiligen Knochenzementes werden absolut steril in die jeweilige Kartusche 20 eingebracht, wenn diese bereits durch den Deckel 36 unter Druckbelastung durch die Druckplatte 33 verschlossen ist. Ein Ausführungsbeispiel, wie dies erreicht werden kann, ist in Fig. 7 dargestellt.

Es befinden sich hier die beiden Komponenten des herzustellenden Knochenzementes in zwei Ampullen 59' und 60', in die steril das flüssige Monomer bzw. die pulvrige Komponente des herzustellenden Knochenzementes eingefüllt wurden. Diese Ampullen sind durch je ein je eine elastomere Membran bildendes Septum 73, 74 verschlossen.

Diese Ampullen oder Behälter 59', 60' können diese Komponenten in den bereits für die Herstellung des betreffenden Knochenzementes erforderlichen, genau abgemessenen Mengen enthalten oder es ist auch möglich, sie im Überschuß darin zu haben und durch mindestens eine Dosiervorrichtung, die dann ebenfalls steril arbeiten muß, abzumessen. In diesem Ausführungsbeispiel ist jedoch angenommen, daß sich diese Komponenten in den vorgesehenen Mengen in diesen Ampullen 59', 60' befinden.

Der Deckel 36, welcher die zugeordnete Kartusche 20 während der Mischphase und vorzugsweise auch während der Ruhephase des in ihr vorzubereitenden Knochenzementes verschließt, weist insgesamt vier Löcher auf, von denen in Fig. 7 zwei zu sehen sind. Jedes solches Loch ist durch ein Septum, wie 87, 88 luftdicht abgesperrt.

Zur sterilen Entnahme der beiden Komponenten aus den Ampullen 59', 60' und deren Überführen in die Kartusche 20 dienen sterilisierte flexible Leitungen 79 bis 82, an deren beiden Enden jeweils eine Kanüle 75 bis 78 und 83 bis 86 angeordnet ist. Die betreffende Bedienungsperson sticht in jedes Septima 73 und 74 je zwei Kanülen 75, 77 bzw. 76, 78 ein, von denen die kürzeren Kanülen 75, 76 der Entnahme der betreffenden Komponenten und die längeren der Entlüftung des Innenraumes der betreffenden Ampulle 59' bzw 60' dienen.

Jede dieser Kanülen 83 bis 86 ist durch ein Septum des Deckels 36 bis in die Kartusche 20 eingestochen, wobei diese Kanülen 83 bis 86 für sie vorgesehene Löcher in der Druckplatte 33 und dem Deckel 36 durchdringen. Zuerst werden die Kanülen 83 bis 86 durch die betreffende Septima, wie 87, 88, des Deckels 36 hindurchgestochen und dann die oberen Kanülen 75 und 76 und danach die Entlüftungskanülen 77 und 78 durch die betreffenden Septima 73, 74 der Ampullen 59', 60' hindurchgestochen und die betreffenden Komponenten fließen nun durch die Leitungen 79 und 80 hindurch in die Kartusche 20 steril ein. Wenn dieser Vorgang beendet ist, zieht die Bedienungsperson die Kanülen 83 bis 86 aus den Septima

11

des Deckels 36, von denen nur die Septima 87 und 88 zu sehen sind, heraus, wobei sich diese Septima wieder luftdicht schließen, und nimmt dann diese Leitungen 79 bis 82 und auch die Ampullen 59´, 60´ von der Haltevorrichtung 61 ab.

Sofort nach dem Einführen der beiden Komponenten in die Kartusche 20 und der Abnahme der Kanülen 83 bis 86 kann mit dem Vermischen der beiden in die Kartusche steril eingefüllten Komponenten begonnen werden, wozu mittels der Welle 31 der nicht dargestellte, das Vermischen dieser Komponenten durchführende Propeller angetrieben wird. Der Propeller 32 kann auch schon bereits während des Einfüllens der beiden Komponenten in Rotation versetzt sein oder erst nach diesem Einfüllen in Rotation gesetzt werden.

Es ist ohne weiteres möglich, diese sterile Einfüllmöglichkeit der Komponenten in die jeweilige Kartusche 20 bei beliebigen Einrichtungen nach der Erfindung vorzusehen, bspw. bei den Maschinen 10 und 10´.

Die Steuervorrichtung 52 kann auch in manchen Fällen als reine Prozeßsteuervorrichtung dienen, indem sie so ausgebildet ist und Signale von mindestens einem Fühler erhält, der auf einen Zustand der in der jeweiligen Kartusche 20 befindlichen, zum Knochenzement vorzubereitende Masse, d.h. der Knochenzementmasse anspricht, welcher prozeßgesteuert werden soll. Dieser oder ein solcher Zustand kann das Fortschreiten des Verrührens oder Polymerisation der Knochenzementmasse sein. Bspw. kann der Zustand der Knochenzementmasse ihre Zähigkeit oder Viskosität sein und durch ein Drehmomentmeßgerät dadurch ermittelt werden, indem dieses Drehmomentmeßgerät das von der Welle 31 zum Antrieb des Propellers 32 übertragene Drehmoment fühlt. Bspw. kann dieses Drehmoment durch die momentane Leistungsaufnahme des Motors 29 unter Division durch dessen Drehzahl oder durch ein es direkt messendes Meßgerät gefühlt werden. Dieses Drehmoment ist abhängig von dem Fortschreiten des Mischvorganges der in der Kartusche 20 befindlichen Knochenzementmasse und dem Fortschreiten von dessen Polymerisationszustand. Wenn bspw. dieses Drehmoment während des Rührens der Knochenzementmasse einen vorbestimmten Wert erreicht, der abhängig von dem Knochenzementtyp und der Temperatur der Knochenzementmasse ist oder sein kann, dann kann dies das Beendigen der Mischphase auslösen und die Ruhephase begonnen werden, d.h. der Propeller 32 wird angehalten. Man kann dann, um auch während der Ruhephase das Fortschreiten der Polymerisation zu ermitteln, vorsehen, während der Ruhephase den Motor 29 von Zeit zu Zeit kurzzeitig mit vorbestimmter, vorzugsweise sehr niedriger, das Polymerisieren nicht störender Drehzahl einzuschalten und das Drehmoment messen und dieses Drehmoment ist abhängig von dem Fortschreiten der Polymerisation. Bspw. kann man alle 30 Sek. oder in anderen vorbestimmten Zeitabständen den Motor 26 kurzzeitig einschalten und das Drehmoment messen, und wenn es dann während der Ruhephase des Knochenzementes einen vorbestimmten Wert überschreitet, der so vorge sehen ist, daß nunmehr der Polymerisationszustand des Knochenzementes den für den Beginn der .Verarbeitungsphase erforderlichen Wertbereich erreicht hat, dann die Ruhephase beendet und die Kartusche mit dem Knochenzement zur Herausnahme freigegeben wird. Oder während der Ruhephase kann der Temperaturanstieg des Knochenzementes gefühlt werden, und wenn er den vorbestimmten Wert gegenüber der Anfangstemperatur zu Beginn der Ruhephase oder zu einem anderen Zeitpunkt überschreitet, der so getroffen ist, daß bei ihm der Polymerisationszustand des Knochenzementes den für den Beginn der Verarbeitung des Knochenzementes erforderlichen Wert des Polymerisationszustandes erreicht hat, wird die Ruhephase selbsttätig beendet und die Kartusche 20 zur Herausnahme aus der Maschine freigegeben.

**Ansprüche**

1. Verfahren zum Vorbereiten von Knochenzement für das nachfolgende Einführen in die der Aufnahme einer Prothese dienende Höhlung eines Knochens, wobei die der Herstellung des Knochenzementes dienenden Komponenten, bei denen es sich um mindestens je eine flüssige und pulverförmige Komponente handelt, in eine Kartusche eingebracht und während einer Mischphase miteinander vermischt werden und anschließend noch eine Ruhephase eingelegt wird, an deren Ende der Polymerisationszustand des Knochenzementes soweit fortgeschritten ist, daß er anschließend verarbeitet werden kann, dadurch gekennzeichnet, daß die Mischphase und/oder die Ruhephase unter Berücksichtigung der Art des jeweiligen Knochenzementes und dessen in die Kartusche eingeführten Mengen seiner Komponenten automatisch programmgesteuert und/oder prozeßgesteuert durchgeführt werden.

2. Verfahren nach Anspruch 1, dadurch gekennzeichnet, daß bei der Programmsteuerung bzw. der Prozeßsteuerung als Parameter die Raumtemperatur des betreffenden Operationssaales und/oder die Umgebungstemperatur der Kartusche und/oder die Temperatur oder die Innentemperatur der Kartusche und/oder die momentane Temperatur des in der Kartusche befindlichen Knochenzements und/oder der Polymerisationszustand des in der Kartusche befindlichen Knochenzementes selbsttätig berücksichtigt werden, wobei erforderlichenfalls, oder falls zweckmäßig, auch noch ein oder mehrere sonstige, die Vorbereitung des Knochenzementes beeinflussende Parameter selbsttätig mit berücksichtigt werden können.

3. Verfahren nach Anspruch 1 oder 2, dadurch gekennzeichnet, daß während der Mischphase und vorzugsweise auch während der Ruhephase die Kartusche zumindest zeitweise gegenüber der Umgebungsatmosphäre abgesperrt und ihrem Innenraum dann zumindest zeitweise, vorzugsweise ständig mittels einer Unterdruckquelle Gas abgesaugt wird, wobei vorzugsweise das abgesaugte Gas durch ein die in ihm enthaltenen Monomere ausfilterndes Aktivkohlefilter hinduchgeleitet wird und/oder vorzugsweise die bei der Aufhebung des durch dieses Absaugen von Gas in der Kartusche entstandenen Unterdruckes in die Kartusche einströmende Luft zuvor ein Sterilfilter zum Ausfiltern von in ihr enthaltenen Bakterien, Krankheitskeimen und dgl. durchströmt.

4. Verfahren nach einem der vorhergehenden Ansprüche, dadurch gekennzeichnet, daß mit der selbsttätigen Steuerung der Mischphase mit dem Einfüllen der Komponenten des betreffenden Knochenzementes in die Kartusche oder erst nach dem Einfüllen dieser Komponenten in die Kartusche begonnen wird, vorzugsweise mit der selbsttätigen Steuerung der Mischphase mit dem Einführen des dem Verrühren der Komponenten des Knochenzementes dienenden Rührorganes in die Kartusche oder nach dem Einführen dieses Rührorganes in die Kartusche begonnen wird.

5. Verfahren nach einem der vorhergehenden Ansprüche, dadurch gekennzeichnet, daß das Mischen der aus den Knochenzementkomponenten bestehenden Masse mittels einer Rührvorrichtung durchgeführt wird, deren rührwirksames Rührorgan, das vorzugsweise ein Propeller ist, für das Mischen vollständig in diese Masse eingetaucht wird und/oder während des Mischens ständig oder zeitweise lageverstellt, vorzugsweise in der Masse auf und ab bewegt wird.

6. Verfahren nach einem der vorhergehenden Ansprüche, dadurch gekennzeichnet, daß die Knochenzementkomponenten zu Beginn der Mischphase auf vorbestimmte niedrige Temperaturen gekühlt sind.

7. Verfahren nach einem der vorhergehenden Ansprüche, dadurch gekennzeichnet, daß die Kartusche während der Mischphase und/oder während der Ruhephase zumindest zeitweise programmgesteuert und/oder prozeßgesteuert erwärmt und/oder gekühlt wird, derart, daß das Fortschreiten des Polymerisierens des Knochenzementes während der Mischphase und/oder während der Ruhephase beeinflußt wird und/oder der Knochenzement am Ende der Ruhephase eine vorbe stimmte Verarbeitungstemperatur hat, die vorzugsweise niedriger als die Raumtemperatur des betreffenden Operationssaales ist.

8. Verfahren nach einem der vorhergehenden Ansprüche, dadurch gekennzeichnet, daß die Kartusche vor der Mischphase in eine ihrem Halten dienende Haltevorrichtung eingebracht und in dieser automatisch festgehalten und erst am Ende der Ruhephase zur vorzugsweise manuellen Entnahme aus der Haltevorrichtung wieder freigegeben wird.

9. Verfahren nach einem der vorhergehenden Ansprüche, dadurch gekennzeichnet, daß die Kartusche vor oder nach dem Einfüllen der pulvrigen Komponente des Knochenzementes durch einen Deckel verschlossen wird, der mindestens eine durch ein Septum verschlossene Öffnung aufweist und mindestens eine Komponente des Knochenzementes mittels einer das oder ein solches Septum durchstechenden Kanüle in die Kartusche eingefüllt wird.

10. Verfahren nach Anspruch 9, dadurch gekennzeichnet, daß mindestens eine Komponente des herzustellenden Knochenzementes, vorzugsweise alle seine Komponenten, in einem durch ein Septum steril verschlossenes Vorratsgefäß bzw. in durch Septima steril verschlossenen Vorratsgefäßen aufbewahrt und mittels einer an beiden Enden mit je einer Kanüle versehenen Leitung bzw. mittels derartigen beidseits mit Kanülen versehenen Leitungen aus diesem Vorratsgefäß bzw. aus diesen Vorratsgefäßen steril entnommen und steril in die betreffende Kartusche eingefüllt wird bzw. werden.

11. Verfahren nach einem der vorhergehenden Ansprüche, dadurch gekennzeichnet, daß die mindestens eine pulvrige Komponente und/oder die mindestens eine flüssige Komponente des herzustellenden Knochenzementes in einem Vorratsgefäß bzw. in Vorratsgefäßen aufbewahrt wird bzw. werden und die in dem betreffenden Vorratsgefäß aufbewahrte Komponente mittels einer an es angeschlossenen Leitung, an der bzw. in der je eine manuell oder durch die Programmsteuervorrichtung selbsttätig einstellbare Dosiervorrichtung vorgesehen ist, in jeweils durch die betreffende Dosiervorrichtung abgemessener Menge in eine Kartusche eingefüllt wird und daß vorzugsweise erst danach in die Kartusche ein Rührorgan eingeführt wird.

12. Verfahren nach einem der vorhergehenden Ansprüche, dadurch gekennzeichnet, daß zumindest Daten, die den jeweils herzustellenden Knochenzement und dessen herzustellende Menge beinhalten und gegebenenfalls noch sonstige Informationen für die Programmsteuervorrichtung bzw. die Prozeßsteuervorrichtung beinhalten können, auf einem Datenträger, vorzugsweise auf der Kartusche oder auf einem die Kartusche aufnehmenden Träger angebracht oder sonstwie gespeichert und durch ein Lesegerät in die Programmsteuervorrichtung bzw. in die Prozeßsteuervorrichtung für die Programmsteuerung bzw. die Prozeßsteuerung eingelesen werden, wobei vorzugsweise der diese Daten tragende Träger ein Einweg-Artikel, vorzugsweise ein steriler Einweg-Artikel ist.

13. Verfahren nach Anspruch 12, dadurch gekennzeichnet, daß der Datenträger in eine vorgesehene, seinem Halten dienende Aufnahme einer Kartuschen-Haltevorrichtung eingesetzt wird.

14. Verfahren nach Anspruch 13, dadurch gekennzeichnet, daß nach dem Einsetzen des Datenträgers in die Kartuschen-Haltevorrichtung eine sterile Kartusche in eine am Datenträger vorgesehene Aufnahme eingesetzt wird, und daß, sofern nicht schon bereits erfolgt, nunmehr die für den herzustellenden Knochenzement erforderlichen Komponenten in diese Kartusche eingefüllt werden und danach ein dem Vermischen der Knochenzementkomponenten miteinander dienendes Rührorgan in die Kartusche eingesetzt und die Kartusche verschlossen, vorzugsweise ihr Innenraum an eine Unterdruckquelle angeschlossen wird.

15. Verfahren nach einem der vorhergehenden Ansprüche, dadurch gekennzeichnet, daß die Drehzahl eines dem Vermischen der Knochenzementkomponenten miteinander dienenden Rührorganes während der Mischpase programmgesteuert und/oder prozeßgesteuert verstellt wird und/oder dieses Rührorgan während dieses Vermischens programmgesteuert und/oder prozeßgesteuert zu Hubbewegungen angetrieben wird, die vorzugsweise, damit sie nicht aus der Knochenzementmasse herausführen, in ihren Hubhöhen auf die jeweils in der Kartusche einzufüllenden oder die in ihr befindliche Menge der Knochenzementkomponenten durch die Programmsteuervorrichtung und/oder durch die Prozeßsteuervorrichtung eingestellt werden und/oder daß die Zeitdauer des Verrührens der Knochenzementkomponenten miteinander und/oder die Zeitdauer der Ruhephase programmgesteuert und/oder prozeßgesteuert wird, wobei der Programmsteuervorrichtung und/oder der Prozeßsteuervorrichtung vorzugsweise jeweils Daten über den betreffenden Knochenzement und über die Menge des jeweils herzustellenden Knochenzementes und vorzugsweise auch der Wert der momentanen Raumtemperatur des Operationssaales geliefert werden und sie unter Verwertung dieser Daten bzw. des momentanen Wertes der Raumtemperatur die Programmsteuerung und/oder die Prozeßsteuerung für die Vorbereitung des jeweiligen Knochenzementes selbsttätig durchführt.

16. Einrichtung zum Vorbereiten von Knochenzement zur Durchführung des Verfahrens nach einem der vorhergehenden Ansprüche, dadurch gekennzeichnet, daß sie eine Programmsteuer- und/oder Prozeßsteuervorrichtung (52) zur automatischen Steuerung der Mischphase und/oder der Ruhephase aufweist.

17. Einrichtung nach Anspruch 16, dadurch gekennzeichnet, daß sie einen Temperaturfühler (58) zum Fühlen der Lufttemperatur der die Einrichtung umgebenden Luft und/oder einen Temperaturfühler (72') zum Fühlen der Wandtemperatur der Kartusche und/oder einen Temperaturfühler zum Fühlen der Innentemperatur der Kartusche, vorzugsweise der Temperatur der in der Kartusche jeweils befindlichen, aus den Komponenten für den Knochenzement bestehenden Masse aufweist.

18. Einrichtung nach Anspruch 16 oder 17, dadurch gekennzeichnet, daß sie eine Unterdruckquelle (47) zum Absaugen von Gas aus der Kartusche (20) und vorzugsweise ein in Reihe mit der Unterdruckquelle angeordnetes Aktivkohlefilter (49) aufweist.

19. Einrichtung nach einem der Ansprüche 16 bis 18, dadurch gekennzeichnet, daß sie eine einen Antriebsmotor (29) und ein Rührorgan (32) aufweisende Rührvorrichtung aufweist, welche vorzugsweise an einem motorisch höhenverstellbaren Träger (27) angeordnet ist.

20. Einrichtung nach Anspruch 19, dadurch gekennzeichnet, daß das Rührorgan während des Rührens in der in der Kartusche befindlichen Knochenzementmasse auf und ab bewegbar ist, und daß die Höhe des Hubs so einstellbar ist, daß das Rührorgan während diesen Auf- und Abbewegungen innerhalb der Knochenzementmasse verbleibt.

21. Einrichtung nach Anspruch 19 oder 20, dadurch gekennzeichnet, daß das Rührorgan mit mindestens zwei unterschiedlichen Drehzahlen antreibbar ist.

22. Einrichtung nach einem der Ansprüche 16 bis 21, dadurch gekennzeichnet, daß sie einen lagever stellbaren Tisch, vorzugsweise einen Drehtisch (15) aufweist, auf welchem die jeweilige Kartusche sich während des Durchführens der Mischphase und der Ruhephase befindet.

23. Einrichtung nach Anspruch 22, dadurch gekennzeichnet, daß sie eine Führung (21), vorzugsweise eine Geradführung aufweist, in welcher ein Schlitten (19) führbar ist, von dem die jeweilige Kartusche getragen wird.

24. Einrichtung nach Anspruch 22 und 23, dadurch gekennzeichnet, daß der lageverstellbare Tisch (15) die Führung (21) aufweist und in eine Aufnahmestellung (Grundstellung) für die Kartusche (20) einstellbar ist, in der die die Kartusche haltende, vorzugsweise sterile Schlitten (19) in die Führung einführbar ist, und daß der Tisch nach dem jeweiligen Einführen eines Schlittens mit Kartusche in eine andere Stellung bewegbar ist, in der der Schlitten zusammen mit der Kartusche gegen Entnahme aus der Führung gesperrt ist und in der die Mischphase und vorzugsweise auch die Ruhephase durchführbar ist.

25. Einrichtung mach einem der Ansprüche 16 bis 24, dadurch gekennzeichnet, daß die Einrichtung eine vorzugsweise oberhalb eines Tisches (15), auf dem die Kartusche sich während der Mischphase und der Ruhephase befindet, angeordnete Halterung (61) für Vorratsgefäße (59, 60; 59', 60') für mindestens eine Komponente des jeweils herzustellenden Knochenzementes aufweist, vorzugsweise für Vorratsgefäße für mindestens je eine pulvrige und flüssige Komponente des herzustellenden Knochenzementes, und daß vorzugweise eine oder mehrere Dosiervorrichtungen (62, 63) an der Einrichtung angeordnet sind, die dem einstellbaren Dosieren einer oder mehrerer der in die Kartusche (20) jeweils einzufüllenden Komponenten des herzustellenden Knochenzementes dient bzw. dienen.

26. Einrichtung nach einem der Ansprüche 16 bis 25, dadurch gekennzeichnet, daß die jeweilige Kartusche (20) durch einen Deckel (36) verschließbar ist, der mindestens eine, vorzugsweise mehrere durch je ein Septima (87, 88) verschlossene Öffnungen aufweist, wobei dieses Septimum bzw. diese Septima dazu dienen, daß in sie mindestens eine Kanüle (83; 84) einstechbar ist, wobei diese Kanüle bzw. diese Kanülen dem Einbringen von pulvrigen und/oder flüssigen Komponenten in die Kartusche zur Herstellung des jeweiligen Knochenzementes und/oder zum Anschließen einer Unterdruckquelle an den Innenraum der Kartusche und/oder zum Anschließen einer Druckausgleichleitung zum Druckausgleich des Innenraumes der Kartusche mit mindestens einem Vorratsgefäß für mindestens eine Knochenzementkomponente dienen.

27. Einrichtung nach einem der Ansprüche 18 bis 26, dadurch gekennzeichnet, daß sie ein Sterilfilter (50) für Luft aufweist, das dem Filtern der in die Kartusche beim Aufheben des Unterdruckes in ihr einströmenden Luft dient.

28. Einrichtung nach einem der vorhergehenden Ansprüche, dadurch gekennzeichnet, daß der jeweiligen Kartusche (20) ein Datenträger (19; 69), vorzugsweise eine Platte zugeordnet ist oder ein Datenträger an ihr angeordnet oder durch sie selbst gebildet ist, welcher Datenträger zumindest einige zur Durchführung der Mischphase und der Ruhephase benötigte Daten trägt oder aufweist, welche Daten vorzugsweise Daten über die Art des jeweils herzustellenden Knochenzementes und über dessen Menge sind, welche Daten durch ein Lesegerät (53) der Einrichtung (10; 10') selbsttätig lesbar und der Programmsteuervorrichtung bzw. der Prozeßsteuervorrichtung zuleitbar sind, vorzugsweise einem Rechner (55), der mit einem Speicher (56) zusammenwirkt, in dem ein Datenfeld oder Kennlinienfeld über den jeweils herzustellenden Knochenzement gespeichert ist, das als Parameter zumindest die Menge des herzustellenden Knochenzementes und mindestens eine durch einen Temperaturfühler zu fühlende, sich auf die Mischphase und die Ruhephase auswirkende Temperatur, vorzugsweise die Raumtemperatur des Operationssaales oder eine Temperatur der Kartusche oder ihres Inhalts, enthält, wobei der Rechner hieraus die im Rahmen der Programmsteuerung bzw. Prozeßsteuerung erforderliche Steuerung der Mischphase und Ruhephase der Vorbereitung des Knochenzementes in vorbestimmter Weise berechnet und entsprechend selbsttätig steuert.

29. Einrichtung nach Anspruch 28, dadurch gekennzeichnet, daß der Datenträger (19) eine Aufnahme (95) zum Halten der Kartusche aufweist.

30. Einrichtung nach einem der Ansprüche 16 bis 29, dadurch gekennzeichnet, daß sie eine Temperiervorrichtung (67; 70, 71, 71'', 90) zum Kühlen und vorzugsweise auch zum Erwärmen der jeweils in der Einrichtung angeordneten Kartusche (20) aufweist, wobei diese Temperiervorrichtung vorzugsweise eine die Kartusche aufnehmende kühlbare und ggfs. beheizbare Kammer aufweist.

31. Einrichtung nach einem der Ansprüche 16 bis 30, dadurch gekennzeichnet, daß sie ein federbelastetes Druckteil, vorzugsweise eine Druckplatte (33) aufweist, die höhenverstellbar angeordnet ist und dem Andrücken eines Deckels (36) an die jeweilge Kartusche (20) dient.

32. Einrichtung nach einem der Ansprüche 16 bis 31, dadurch gekennzeichnet, die Kartusche (20) und/oder der Datenträger (19; 69) und/oder das dem Vermischen der Knochenzementkomponenten dienende Rührorgan (32) vorzugsweise zusammen mit der es tragenden Welle (31) und/oder der dem Verschließen der Kartusche (20) während der Mischphase und vorzugsweise auch während der Ruhephase dienende Deckel (36) sterilisierbare Einweg-Artikel sind.

Fig.1

Fig. 3

Fig. 2

Fig. 4

Fig. 5

Fig. 6

Fig. 7

<table>
<tr><td colspan="2"></td></tr>
</table>

| | | | |
|---|---|---|---|

<table>
<thead>
<tr><th></th><th colspan="2"></th></tr>
</thead>
</table>

## EUROPÄISCHER RECHERCHENBERICHT

**Europäisches Patentamt**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 290 746  (ALLO PRO) <br> * Ganzes Dokument * <br> --- | 1-32 | B 01 F  15/00 <br> B 01 F  13/06 |
| A | US-A-4 020 154  (PERLA) <br> * Fig.; Zusammenfassung * <br> --- | 3 | |
| A | DE-U-8 606 633  (WEISS) <br> * Patentanspruch 1; Fig.; Seite 10, Zeilen 6-9 * <br> --- | 19,20, 22-24 | |
| A | DE-B-1 296 956  (QUECK) <br> * Spalte 3, Zeilen 41-49; Fig. * <br> --- | 25 | |
| A | US-A-3 718 069  (PLOMER) <br> --- | | |
| A | FR-A-1 100 019  (JAVY) <br> --- | | |
| A | CH-A-  438 223  (SPANGENBERG) <br> --- | | |
| A | BE-A-  631 264  (AMPSIN) <br> --- | | |
| A | DE-A-3 519 713  (SCHMIDT) <br> --- | | |
| A | US-A-3 514 080  (PRICE) <br> --- | | |
| A | EP-A-0 246 394  (LAEMPE) <br> --- | | |
| A | US-A-4 042 221  (MYERS) <br> ----- | | |

**RECHERCHIERTE SACHGEBIETE (Int. Cl.5)**

B 01 F
A 61 F

Der vorliegende Recherchenbericht wurde für alle Patentansprüche erstellt

| Recherchenort | Abschlußdatum der Recherche | Prüfer |
|---|---|---|
| DEN HAAG | 18-09-1990 | PEETERS S. |